# EUROPEAN PATENT APPLICATION

(11) **EP 2 965 692 A1**
(43) Date of publication of application: **13.01.2016**
(21) Application number: 15382185.5
(22) Date of filing: 17.04.2015
(51) Int. Cl.: A61B 5/16, A61B 5/00

(54) **A SYSTEM AND METHOD FOR COGNITIVE AND SENSORY STIMULATION**

(30) Priority: 11.07.2014 ES 201431057 P
(71) Applicant: Neurobai, S.L., 48011 Bilbao (ES)
(72) Inventor: Garrues Remirez, Joaquin Luis, 48007 Vizcaya (ES)
(74) Representative: Isern-Jara, Nuria

(57) **Abstract**

A system and method for cognitive and sensory stimulation, which facilitates the personalised and coordinated stimulation of users at a sensory and cognitive level, in order to prevent any type of dementia or pathology, or any type of dementia developed with normal or pathological ageing, said system comprising a processing unit in data communication with display means, at least one user interface and sensory stimulation means, the processing unit being configured to store and manage a plurality of operating sequences from the sensory stimulation means based on a number of signals received from the user interface and cognitive and/or sensory interaction with a user, in which the sensory stimulation means are configured to stimulate at least one visual, tactile, olfactory or sound field, or a combination thereof, of the user.

## Description

### OBJECT OF THE INVENTION

The present application seeks to register a system and method for cognitive and sensory stimulation, which includes significant innovations.

More specifically, the invention proposes the development of a system and method followed by the system, which facilitates the personalised and coordinated stimulation of users at a sensory and cognitive level, in order to prevent any type of dementia or pathology, or any type of dementia developed with normal or pathological ageing.

### BACKGROUND OF THE INVENTION

Psychotropic medicines that are commonly used to reduce the frequency and severity of the behavioural symptoms of some pathologies, especially dementia or chronic pain, are well-known. However, the efficacy thereof has proved to be limited, whilst also producing frequent, and sometimes dangerous, side effects. As a result of all this, over the past few years, non-pharmacological interventions have gained relevance in the treatment of cognitive and emotional problems in people suffering from different physical and mental disorders. This is due to the discrete effectiveness thereof, as shown in the reduction and control of cognitive and behaviour problems caused by various pathologies, such as dementia, stress or chronic pain.

Over the past twenty years, different non-pharmacological interventions have started to be tested. Cognitive simulation and cognitive rehabilitation are potentially the most well-known examples within the Clinical Psychology field. These techniques aim to improve the function of a number of cognitive processes (memory, attention, concentration and executive functions), without evaluating the action mechanisms that trigger said changes. However, throughout the past decade, neuroimaging studies (PET, MRI) have been able to objectively establish the effectiveness of cognitive stimulation therapy (CST) by measuring volumes and the activation of structures related to higher-level cognitive activity.

Traditionally, CST programmes consisted of stimulating different mental process based on the premise that in this way, the level of brain plasticity improves, thus slowing cognitive decline. The high expectations of these treatments explain the meta-analysis studies carried out, under which the efficiency, effectiveness and cost-effectiveness thereof is estimated. One example is the prestigious work published by The Cochrane Collaboration (Woods, Aguirre and Orrell, 2012) which, based on an extensive review of 15 studies that comply the maximum methodological requirements, having involved a total of 718 participants (407 receiving cognitive stimulation and 311 in the control group), concludes that CST produces beneficial effects for people with mild or moderate cognitive deterioration, exceeding the effects of any medication.

These results, as well as others before, favour the design of different structured methods and tasks through which the level of activity is maintained. However, fundamentally owing to the cost thereof, said therapies are carried out in a group format, following standard rules that neglect the interests, motivations and preferences of each individual. This explains why, in some cases, the user may show signs of nervousness and frustration with routine tasks, which are often boring and bear very little resemblance to everyday life. These pen and paper exercises often evoke negative memories of childhood school experiences or negative memories of another nature and may engender a competitive spirit that adds more stress to the situation.

Another limitation of CST is that it requires the ability to communicate. This latter limitation usually comes about naturally as a result of the ageing process in the brain. A study carried out by Murphy, Gray and Cox (2007) mentions how the majority of clinical studies do not take the gradual deterioration of communicative abilities into account, thus giving rise to significant "distress" in users and carers. When geriatric diseases progress, people become less accessible in terms of their interactions, it thus being especially difficult to evaluate their opinions and desires.

Hyperstimulation or psychostimulation therapies, which form part of overstimulation programmes for elderly people, do not guarantee an improvement in quality of life for everyone with problems or the reduction of the number of elderly people who are aware of their ability loss. In any case, highly structured cognitive stimulation therapies, such as CST, are used for people affected by mild cognitive deterioration and people in the initial phases of dementia. There are other people who do not benefit from CST, who may receive other non-pharmacological treatments such as Multisensory therapy (MST).

MSTs were introduced in 1969 by Ayres, in order to explain the relationship between sensory processing and behaviour. Many children and adults with sensory integration dysfunction, as well as people affected by dementia, develop a tendency to reject multiple sensory challenges linked to cognitive hyperstimulation, and respond with signs of rejection or "tantrums" when they are forced to do tasks (Case-Smith, 1996).

From the MST perspective, it is important to expose individuals to sensory information derived from the environment progressively, in order to prevent the appearance of certain episodes and cognitive and behavioural symptoms. One of the features that differentiates MST from hyperstimulation techniques (CST) that do not form part of the therapeutic knowledge of nursing homes and day centres, is its guiding nature, i.e. the user is encouraged to choose the most appropriate sensory stimuli according to their preferences. Furthermore, the stimuli are not sequential. Lastly, sustained stimulation techniques, where different sensory modalities are offered, appear to be more accessible to all users with no distinction between their intellectual levels. In contrast, reality- and memory-oriented psychostimulation therapies, which form part of the hyperstimulation protocols, only appear to benefit healthy people and users in the first stages of dementia.

With regards to MST, a recent study published by a North American government agency ("A Sistematic Evidence Review Non-pharmacological Interventions for Behavioral Symptoms of Dementia. Department of Veterans Affair Health Services Research & Veterans Affair Health Services Research") published in 2011, highlighted the benefits of music, touch and olfactory stimuli for treating certain problems such as states of agitation or confusion.

Based on the foregoing, electronic devices that try to stimulate users cognitively (CST) or through senses (MST) have been developed, although they act independently rather than being coordinated and present the aforementioned limitations.

Moreover, devices that provide a cognitive or sensory evaluation (diagnosis) of a subject are also known about. In order to perform this task, a series of sensory stimuli may be presented to the subject and their physiological signals when presented with a series of stimuli are collected. The physiological data may be processed to generate a set of information that includes one or more quantitative values linked to the category of a profile, i.e., this type of known devices are designed to obtain a diagnosis of the user by measuring physiological data produced when faced with a series of stimuli, such that it is necessary for staff with specific medical training to be present and supervise. The users themselves do not improve at all; rather the pathology they suffer from is simply determined, and the severity thereof, known, it being possible to repeat the same tests over time, in order to observe the evolution of the pathology. Furthermore, obtaining physiological signals from the user implies applying sensors such as electrodes to them during a certain period of time, which causes added tension for the user, thus causing them to produce a negative response.

### DESCRIPTION OF THE INVENTION

The present invention has been developed for the purpose of providing a system and method for cognitive and sensory stimulation that solves the aforementioned drawbacks, further contributing other additional advantages that shall become evident as of the description included below.

An object of the present invention is therefore a system and method for cognitive and sensory stimulation, comprising a processing unit in data communication with display means, at least one user interface and sensory stimulation means, the processing unit being configured to store and manage a plurality of operating sequences from the sensory stimulation means based on a number of signals received from the user interface and cognitive and/or sensory interaction with a user, in which the sensory stimulation means are configured to stimulate at least one visual, tactile, olfactory or sound field, or a combination thereof, of the user.

Together, these features successfully produce a system that enables a user to be stimulated cognitively and through senses within their own cognitive and sensory thresholds. Said stimulation is managed simultaneously by the processing unit and does not require the use of sensors and/or electrodes on the user, therefore meaning they are not subjected to detrimental tension. Based on the users themselves, the type and level of stimulation that the system carries out is adjusted through the interface at a cognitive level, as are the sensory stimulation means. This therefore constitutes a difference in the way in which the invention works and the process in the state of the art, where the type and level of stimulation are pre-set.

The system is based on the use of electronic means and low-difficulty and easy accessible interfaces that achieve greater interaction between the person and the stimulus. All of this has proven to be especially useful for people with a greater level of dependence, reduced mobility and communication ability. The use of new technologies facilitates an attractive stimulation presentation format, in addition to making it possible to coordinate two formats, for example audio and visual, in such a way that people who are affected by one of these senses do not suffer; they can offer quick information on the level of success of the task, thus enabling the person to correct some strategies thanks to user interaction with the processing unit through the user interface. The system provides a safe and non-threatening multisensory environment, which is the case with known sensory environments. This environment aids user interaction whilst carrying out the cognitive stimulation exercises.

It must be noted that the present invention is to be used subsequent to a diagnosis made by medical staff and does not require staff presence and/or supervision from people with specific medical training, as is the case in the state of the art.

In accordance with one feature of the invention, the display means and user interface may be integrated within the same touchscreen, which may further be arranged in a table structure. In this way, the user may easily interact with the system, being able to sit down and do the cognitive exercises without the risk of falling.

Preferably, the sensory stimulation means may comprise a variety of components. One example of visual stimulation means would be a water column linked to at least one bubble generator, to a first coloured light emitter and a first button panel, it thus being possible to stimulate the user visually. The user may operate said first button panel in order to vary the activation of the different colours in the first light emitter if they so wish. In the present description, it shall be understood that any fluid appropriate for the same purpose may be used in the column in place of water.

The stimulation means may furthermore comprise a second light emitter for emitting different coloured light beams for visual stimulation, said second light emitter being linked to a second button panel, which may also be operated by the user.

The sensory stimulation means may furthermore comprise at least one long, flexible element for visual stimulation, which is configured to emit light and is arranged on a surface where the user is positioned.

For the purposes of visual simulation, the sensory stimulation means may furthermore comprise at least one main projector of changeable visual effects to be projected onto at least one surface.

The sensory stimulation means may furthermore comprise a light-up scale linked to at least one microphone for sound and visual stimulation. This link may be established through the processing unit which, depending on the sound volume emitted by the user through the microphone, will light up a greater or lesser number of portions on the scale.

The sensory stimulation means may furthermore comprise at least one aroma diffuser for olfactory stimulation.

The sensory stimulation means may furthermore comprise a number of sound emitting means for sound stimulation.

The sensory stimulation means may furthermore comprise a vibrating water bed for touch stimulation, the same being linked to at least one water heater so that the water is always at the same temperature. This vibrating water bed may operate in coordination with the sound emitting means, given the action of the processing unit, through which sound stimulation is meanwhile provided.

Another object of the invention is a method for cognitive and sensory stimulation for use with the system previously described, comprising the stages of:
a) Entering external qualification data through the user interface;
b) linking the external qualification data to a plurality of indicators through the processing unit;
c) quantifying the external qualification data for each indicator through the processing unit;
d) determining the operating sequence of the sensory stimulation means in accordance with the previous stages through the processing unit and by activating the sensory stimulation means; and
e) determining the cognitive stimulation exercises to be carried out by the user through the processing unit and activating the user interface in order to carry out the cognitive stimulation exercises.

In the present application, it is understood that the external qualification data constitutes data originating from external tests, the same having been sufficiently checked and validated (such as tests for example), which are commonly used in the field of application. By way of example, these tests may be a test that has been used to evaluate the user; entering this qualification data does not imply under any circumstances the study and/or interpretation thereof.

As a result of these features, it is possible to develop a method to be applied to the above system, in which it is possible to administer sensory stimulation according to certain parameters (frequency, intensity, duration, level of difficulty) as well as cognitive stimulation adapted to the exclusive and particular needs of the user, without causing overstimulation or inappropriate stimulation. In order to put the method into practice, the user's preferences, cognitive level and sensory level shall be taken into account, and not vice versa. Furthermore, the stimulation of certain sensory components, such as the auditory or olfactory senses, is facilitated, which is highly important as evidenced in different studies.

In accordance with one aspect of the invention, in stage b) the indicators may be at least one of a cognitive level, sensory state, functional level, behavioural level, level of awareness and level of consciousness or a combination thereof.

In order to carry out stage d), at least one of the behavioural level, awareness level, consciousness level and sensory state indicators, or a combination thereof, is taken into account. The sensory state determines the state of the sensory channels (if the user can see, hear, feel and perceive, they can respond).

As for carrying out stage e), at least one of the indicators of cognitive level and functional level or a combination thereof is taken into account.

In some cases, the operation of at least one of the sensory stimulation means and the activation of the user interface to carry out the cognitive stimulation exercises may be carried out together and in a coordinated manner or sequentially.

The present method for cognitive and sensory stimulation furthermore comprises storing a set of execution data obtained after having completed stages d) and e) and linking said execution data to a user. This feature makes it possible to continuously register the tasks and, if applicable, adapt them appropriately to the exogenous indicators and their score level (menu), i.e., to personal style.

Other characteristics and advantages of the present system for cognitive and sensory stimulation and the subsequent method, object of the present invention, will become clear in light of the description of a preferred, though non-exclusive, embodiment, which, by way of a non-limiting example, is illustrated in the accompanying drawings, wherein:

### BRIEF DESCRIPTION OF THE DRAWINGS

Figure 1.- Is a schematic perspective view of a system according to an object of the present invention applied in a room; and
Figure 2.- Is a graph based on the results produced by using the system shown in figure 1 over time.

### DESCRIPTION OF A PREFERRED EMBODIMENT

In light of the aforementioned figures, and in accordance with the numbering adopted, it is possible to observe a preferred embodiment of the invention, which comprises the parts and elements indicated and described in detail below.

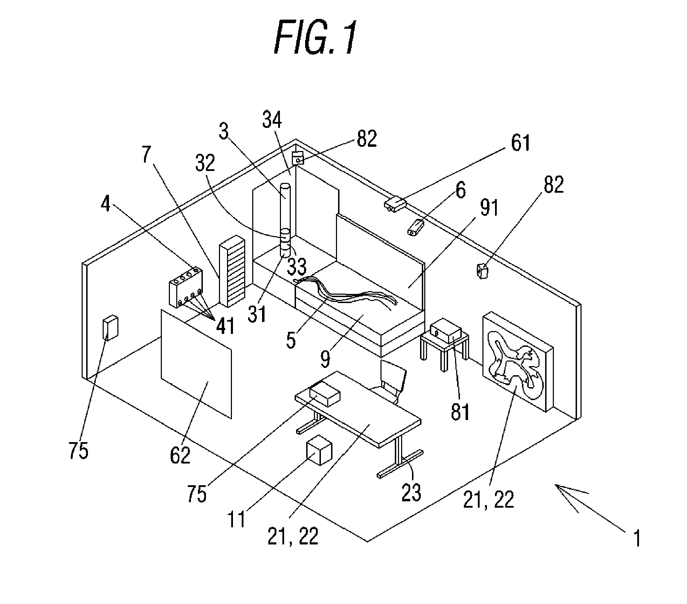

In figure 1, it is possible to observe how the present system for cognitive and sensory stimulation 1 comprises a processing unit 11 in data communication with display means 21, at least one user interface 22 and sensory stimulation means. The processing unit 11 is configured to store and manage a plurality of operating sequences from the sensory stimulation means based on a number of signals received from the user interface 22 and the cognitive and/or sensory interaction with a user, through the sensory stimulation means for example, in which the sensory stimulation means are configured to stimulate at least one visual, tactile, olfactory or sound field of the user or a combination thereof. Preferably, the display means 21 and the user interface 22 are integrated into the same touchscreen and the touchscreen is arranged in a table structure 23. However, it is clear to a person skilled in the art that this arrangement may be modified depending on the particular needs of each case. In figure 1, it is possible to observe that a second touchscreen with display means 21 has been provided, in addition to a user interface 22 situated on a wall or similar. Using these display means 21 on the wall, the user may interact with the images produced, by touching a specific colour or shape with their hand or with a pencil for example.

Continuing with figure 1, it is possible to observe that the sensory stimulation means comprise a water column 3 linked to a bubble generator 31, a first coloured light emitter 32 and a first button panel 33, it being possible for this button panel 33 to be positioned on the water column 3 itself or on a remote controller (not shown). Any other type of appropriate fluid for this purpose may be used instead of water.

In the present embodiment of the water column 3, the bubbles emitted by the bubble generator 31 are lit up with different colours as a result of a fourth group of LEDs of the first coloured light emitter 32, for example, the same being directed towards the area where the bubbles are generated. These colours may be blue, red, green and/or yellow. The plurality of LEDs follow a sequence, progressively lighting up and turning off. That is to say, the brightness of the LEDs corresponds to a sensorial function. All of this is managed via the processing unit 11.

The first button panel 33 preferably comprises several buttons that correspond to several different colours. Whenever any of them is pressed, the selected colour turns on and the others turn off. At the same time, the button that has been pressed lights up. The LED of the colour selected remains lit up for a certain amount of time, before the sequential process managed by the processing unit 11 is restarted. The selected switch also turns off when the cycle starts again or when another switch on the first button panel 33 is pressed. In order to increase visual sensation, the water column 3 may be positioned close to reflective surfaces 34 such as mirrors.

The stimulation means furthermore comprise a second light emitter 4 configured to emit different coloured light beams, said second light emitter 4 being linked to a second button panel 41. This second button panel 41 may also be positioned on a remote control (not shown). The operating sequence is managed by the processing unit 11 or may be interrupted when the user actuates any switch of the second button panel 41, the same subsequently continuing the sequence indicated by the processing unit 11.

The sensory stimulation means furthermore comprise at least one long, flexible element, configured to emit light; it is preferably a plurality of optical fibres 5 arranged on a surface where the user is positioned. This plurality of optical fibres 5 is linked in data communication to the processing unit 11, which indicates the operating sequence, such as the colour of said optical fibres 5 and duration, intensity or frequency, for example. Just like the water column 3, the user may actuate these optical fibres 5 at specific time intervals using a remote control (not shown).

The sensory stimulation means furthermore comprise at least one main projector 6 of changeable visual effects on at least one surface of the room, such as a wall, the floor or ceiling, for example. The shapes that these visual effects may take on may have a liquid appearance. The installation of a secondary projector 61 for emitting relaxing images on a screen 62 may also be provided. Indeed, studies carried out on the effect and impact the main projector 6 has on the person being treated prove that stimulation in response to a changing image relaxes the individual's rale.

The sensory stimulation means may furthermore comprise a light-up scale 7 linked to at least one microphone (not shown). When the user emits a sound into the microphone, the scale 7 lights up in levels depending on the volume of the sound. Furthermore, the different levels may be different colours so that the user can perceive them better.

In the present embodiment, the sensory stimulation means furthermore comprise a pair of aroma diffusers 75. One of them is preferably situated on the table structure 23 and the another, for example, on a wall of the room where the cognitive and sensory stimulation system 1 is situated. Each aroma diffuser 75 comprises an ultrasound module (not shown) linked to a number of essential oils mixed with water for example; a fan (not shown) it responsible for spreading the aroma into the environment or a mask (not shown) may be positioned on the users face.

The use of the aroma diffuser 75 entails a reduction in behavioural problems, lowers aggression, increases social contact, improves linguistic function and states of confusion, improves reality orientation, reduces pain and levels of depression.

The sensory stimulation means furthermore comprise sound emitting means; these sound emitting means may comprise an emitter/amplifier 81 linked to the processing unit 11 and a number of loud speakers 82 arranged around the room. These sound emitting means may function in a coordinated manner with the vibrating water bed 9 advantageously linked to a water heater (not shown) to maintain the water at a constant temperature and thus ensure a more comfortable environment; in this regard, soft protection 91 may be arranged on the walls of the room to prevent the user from hurting themselves. The vibration of the water bed 9 is produced to the rhythm of music. As may be seen in figure 1, a plurality of optical fibres 5 are situated on this water bed along with the user.

The use of sound emitting means reduces agitation, irritability, depression and anxiety and improves mood, orientation and facial recognition.

In order for the system for cognitive and sensory stimulation 1 to function, a method for cognitive and sensory stimulation must be carried out, the same comprising the stages of:
a) Entering external qualification data into the user interface. As mentioned above, this external qualification data may come from tests that have already been carried out and checked. The data may be entered, for example, through the display means 21 and the user interface 22 or telematically between servers, external memory storage means (not shown) and any other similar means;
b) linking the external qualification data to a plurality of indicators through the processing unit 11 by means of running a computer programme. There are preferably six indicators, although a person skilled in the art can change said number depending on particular needs. The indicators show cognitive level, sensory level, behaviour level, level of awareness and level of consciousness or a combination thereof. Within the use of value-based levels of the person's state, these six variables are preferably used, more potentially being used in the future and these variables, collected in different scientifically validated tests, give us a current picture of the individual. These six indicators are defined in the following way:
   - Cognitive state: this is related to the ability to understand and rationalise a specific reality.
   - Functional level: this is related to an individual's ability to relate to the world around them to achieve their specific objectives, get dressed, eat.
   - Behavioural level: this is related to the state of anxiety, depression, apathy, etc.
   - Awareness and consciousness: these indicators indicate what comprises a dream or a dream state and differentiates between a reversible and non-reversible somatic state.
   - The sensory state determines the state of the sensory channels (if the user can see, hear, feel, perceive, they can respond).
c) quantifying the external qualification data for each indicator through the processing unit 11. After linking to an indicator, the external qualification data is quantified; in the present case four are considered: mild, moderate, severe or very severe, although the classification will obviously be modified in accordance with the particular needs of each case. By way of example, if in the tests to obtain external data a field is valued at 75%, the linked indicator is quantified as severe and if it is valued with 100%, the linked indicator is quantified as very severe;
d) determining the operating sequence of the sensory stimulation means according to the previous stages through the processing unit 11 and activating the sensory stimulation means. Based on behavioural level, level of awareness, level of consciousness and the sensory state (the effectiveness of the sensory channels: deaf, blind, etc.) indicators, the processing unit 11 determine which sensory stimulation means will work, as well as the operational variables such as the sequence, duration or intensity of the same, for example. If, for example, the processing unit 11 determines that the user has a visual or auditory impairment, it activates the sensory stimulation means that do not stimulate these senses; and
e) determining the cognitive stimulation exercises to be carried out by the user through the processing unit 11 and activating the user interface 22 to carry out the cognitive stimulation exercises. Based on the cognitive level and functional level indicators, the processing unit 11 determines which cognitive stimulation exercises are carried out, for example, on a table structure 23. These cognitive exercises work on all spheres of cognitive stimulation such as attention, concentration and perception, episodic memory, calculation, etc. Depending on the quantification of the cognitive level and functional level indicators, the unit may determine that the user is unable to carry out all of the cognitive stimulation exercises.

In accordance with one feature of the present method, the operation of at least one of the sensory stimulation means and the activation of the user interface (22) to carry out cognitive stimulation exercises is done together in a coordinated manner. The processing unit 11, depending on the stages a), b) and c), may determine the way in which some of the sensory stimulation means operate together and in coordination and the realisation of cognitive stimulation exercises. An example may be the sensory stimulation with the aroma diffuser 75 situated on the touchscreen with the user interface 22 and the display means 21; the exercise may consist of exposing the user to a specific aroma or sequence of aromas emitted by the aroma diffuser 75 and subsequently recording these aromas, entering their response into the cognitive stimulation exercise being carried out. Another example may be emitting a sound through the sound emitting means and the user must recognise and associate them, entering their answer in the cognitive stimulation exercise being carried out.

The present method for cognitive and sensory stimulation furthermore comprises storing a set of execution data obtained after carrying out stages d) and e) and linking said execution data to a user through the processing unit 11.

Exceptionally, occasional orders may be entered through the user interface 22 to execute a sensory or cognitive stimulus not provided by the processing unit 11.

The use of the present system and the method for using the same makes it possible to increase the user's level of activation and consciousness and provokes significant behavioural responses. The scientific basis of this system and method postulates that frequent and varied exposure to sensory stimuli aids both dendrite growth and the improvement of connections in damaged nervous systems.

When the method that regulates the operation of the present invention is carried out, depending on the functional and behavioural cognitive level, specifically from stage a) - b) where the information is entered and stimulation is determined, the user may relax due to the sensory stimulation that suits their needs, and subsequently carry out the cognitive stimulation exercises in a more relaxed and positive state, thus ensuring it is beneficial for the user.

In short, the ultimate objective of the multisensory environments created by the system is to create enriched atmospheres that aid the recovery of the user's nervous system.

These multisensory environments of the system have been designed to aid relaxation, provide an enjoyable experience and inhibit behavioural changes (Hotz, et al. 1996). When they have been used with different population groups, such as children with severe brain damage, significant changes in physiological measurements have been shown: a lower heart rate, muscle tone, and agitation behavioural levels.

In practice, when the user has cognitive deficits, the processing unit 11 determines active work in the sensory field given their cognitive limitations, for example the presence of the scale 7. Access to the underlying benefits of using this scale 7, is motivated by a low-medium quantification / score at the cognitive level, which prevents active work over the entire cognitive spectrum that comprises the user interface 22 and the display means 21 in the table structure 23, and in its absence, the use of this device achieves an active sensory stimulation. If, on the contrary, the user were to have a high quantification at the cognitive level, a passive sensory stimulation would be produced along with the execution of cognitive exercises.

When the individual is cognitively optimal and in the other behavioural indicators they are anxious, depressed, distressed or stressed, the processing unit 11 determines the operating variables of the sensory stimulation means, for example a specific light, music or smell that is appropriate to their behavioural level. As cognitive level decreases, exposure to sensory stimuli is more active and the spectrum of cognitive exercises is limited by the cognitive deficit that the processing unit 11 of this entire method determines.

Continuing with the examples, if the user has a high level of anxiety, the water bed 9 does not vibrate and the brightness of the optical fibres 5 are warmer colours, a relaxing aroma is selected that is appropriate for emitting to the user and if they are physically in a wheelchair, they cannot lie down.

Regarding the cognitive stimulation exercises proposed by the present method and carried out through the present system, they comprise a set of strategies and processes aimed at improving the activity of different cognitive abilities and functions, such as visual, auditory and olfactory perception, attention, rationalisation, short- and long-term memory, reality orientation, calculation, executive functions and praxis. In order to do so, different activities with a dual purpose, i.e. of stimulating mental abilities and improving the functionality thereof, are selected.

The following exemplary actuation and intervention protocol are presented in the cognitive stimulation exercises, in which the following tasks are described:
1) Activities aimed at orientation in time and space:
   Aim: that the person is able to identify time-space cues that enable them to situate themselves with familiarity in a certain moment of time and place.
   i) Recognise the date, time, day of the week, associate dates with something important in that person's life or community.
   ii) Recognise where they are, their city, etc.
   iii) Biographical information: their biographic profile, place of birth, etc.
2) Memory activities:
   Objective: stimulate the encoding, storage and recovery processes of certain types of sensory memory, both short- and long-term.
   i) Short-term auditory memory stimulation tasks: telephone numbers, people's names or supermarket products.
   ii) Long-term auditory memory stimulation tasks: learning products from the shopping list, stories or relevant headlines that appear in the news.
   iii) Short-term visual memory stimulation tasks: remembering objects that are on the shelf or the clothing worn by people present in the room.
   iv) Short-term visual memory stimulation tasks: recalling objects that are in their room.
   v) Recognition tasks: recognising similarities and difference, linking two images.
   vi) Autobiographical memory tasks: family dates and history.
3) Perception and recognition tasks:
   Aims: improving the level of recognition of different sensory modalities.
   vii) Visual recognition: identifying strange stimuli in a list of images, crossing out certain shapes or letters in sets whilst looking for the one that stands out.
   i) Facial recognition: family, famous people.
   ii) Recognition of a body diagram: identifying different parts of the body and checking if it follows a logical order, e.g. head, body and extremities.
   iii) Object recognition by touch: rough, smooth, cylindrical.
   iv) Familiar route recognition.
   v) Recognition of visual cues: left-right, up-down.
4) Executive activity tasks.
   Aim: to analyse sustained and divided attention capacity, concentration, sequencing and behavioural inhibition, planning and fluency capacity and cognitive flexibility.
   i) Sequence in a set order: following a set of logical criteria, for example, spatial: home (entrance hall, lift, door, hallway...), numbers (1, 2, ...), letters.
   ii) Reverse order: working memory in reverse order HOUSE: ESUOH; 5-4-3-2-1.
   iii) Stimuli interference that is presented simultaneously and from which only one must be identified.
   iv) Reproducing auditory rhythms.
   v) Link cues: door numbers with houses, numbers with pictures, etc.
5) Praxia tasks.
   Aim: analyse the ability to carry out simple or complex conscious motor sequences.
   i) Visual-construction: Reproducing images.
   ii) Drawing: Analysing the order followed when producing the drawing.
   iii) Building models from a variety of materials.
   iv) Ability to carry out acts related to food (hold a fork), hygiene (brush hair), etc. v) Ability to imitate models: gestural actions, greetings, reproducing actions in front of a mirror.
6) Language tasks:
   Aim: To improve expressive, comprehensive, nominal, reading and writing skills.
   i) Spontaneous language and verbal fluency exercises.
   ii) Determining the capacity to name objects.
   iii) Command of repeated language.
   iv) Ability to read and write.
   v) Ability to identify the semantic categories of a variety of nouns and adjectives.
   vi) Verbal fluency: practising the ability to reproduce words according to a criterion.
   vii)Reading and writing tasks.

In order to check the effectiveness of the present system and method for executing the same, a study has been carried out, taking a sample of 30 users that live in nursing homes. The experimental group has completed a year using the present system, whilst the control group has continued with their usual activities at the centre. The results show a clear tendency for the users of the experimental group to improve in comparison to the control group.

### 1) Methodology

### 1.1) Sample

The sample is made up of 30 users, 15 of whom formed the experimental group and 15 of whom were placed in the control group. The features of the sample are presented below:

| | EXPERIMENTAL GROUP | CONTROL GROUP |
|---|---|---|
| No. of subjects | 15 | 15 |
| Gender (female) | 12 (80%) | 7 (47%) |
| Age | 83.8±5.7 | 83.3±6.9 |
| Initial MMSE score | 17.4±4.7 | 21.9±6.1 |

The participation criteria were as follows: score higher than 11 in the MMSE, to have communication capacity, absence of auditory, visual and olfactory sensory deficits, acceptance of the study conditions and not having psychiatric symptoms that may affect the test.

The exclusion criteria were: traumatic brain damage, intercranial pathology (subdural hematoma; hydrocephalus), nutritional-metabolic deficit (B12 vitamin, endocrine disruption, chronic alcoholism, drug abuse, kidney failure or infections of the central nervous system) or infectious organisms (HIV, syphilis or herpes simplex).

There is no significant difference in the proportions of men and women (p = 0.128; Fischer's exact test). Regarding age, there are no significant differences (p = 0.870; Mann-Whitney U test), nor between the initial scores obtained (p = 0.067; Mann-Whitney U test).

### 1.2) Variables.

The variables analysed in the study were:
- Mental state through the measurement of time and space orientation, memory registration, attention and calculation, recall, language and constructive praxis.

### 1.3) Evaluation Tools

Lobo's "Mini-mental State Examination" was used, which is an adapted and validated version of the MMSE (Folstein et al., 1975) in Spain since it is a brief and simple screening test on cognitive performance and it is widely used. It evaluates orientation, information registration, attention, calculation, recall, language and construction.

### 1.4) Method

Firstly, the sample was selected. In order to do so, it was decided that 30 users would be chosen, men and women over the age of 65 with a prior diagnosis of mild or moderate dementia. Before starting the study, the first screening test was carried out. Then, the sessions for using the present system and method for executing the same were scheduled for the participants of the experimental group, who carried out one session per week for a year. Said routine firstly consisted of 20 minutes of sensory stimulation in which the participants, either lying on the water bed 9 or sitting in a wheelchair, had both the optical fibres 5 and the remote controllers at their disposal, with which they could interact. In turn, the water column 3 was activated, as well as the main projector 6 and the sound emitting means. Lastly, an aroma diffuser 75 and the microphone linked to the scale 7 also formed part of the stimulatory field of the elderly. The total duration was 20 minutes. After this time, the cognitive exercises corresponding to the following areas were carried out:
- Visual perception.
- Auditory perception.
- Olfactory perception.
- Memory.
- Attention.
- Calculation.
- Praxia.
- Emotional intelligence.

Furthermore, the control group benefited from sensory therapy, continuing with the daily activities at the centre together with the experimental group.

### 1.5) Statistical Analysis

The group the system was applied to was formed of 15 users and there were 15 in the control group.

Regarding the results, a qualitative analysis was carried out, dividing the users into two groups depending on whether the user improved or not. In order to do so, the differences in scores between the different measurements were calculated, classifying individuals with no difference in scores (0 in the comparisons) as "No improvement". The analysis was carried out via the use of SPSS v19 software. The significance test was Fisher's exact test.

### 2) Results

### 2.1) Measurement obtained

The raw results obtained for the experimental group and the control group may be seen in the following tables:
Experimental group

| Subject | Age | Gender | Start | Six months | Twelve months |
|---|---|---|---|---|---|
| 1 | 80 | m | 22 | 20 | 19 |
| 2 | 96 | f | 18 | 18 | 20 |
| 3 | 77 | f | 20 | 21 | 22 |
| 4 | 77 | f | 12 | 9 | 6 |
| 5 | 91 | f | 28 | 29 | 31 |
| 6 | 83 | f | 11 | 11 | 12 |
| 7 | 90 | f | 18 | 18 | 18 |
| 8 | 83 | f | 13 | 13 | 14 |
| 9 | 84 | f | 16 | 17 | 18 |
| 10 | 78 | m | 23 | 23 | 25 |
| 11 | 86 | m | 11 | 11 | 15 |
| 12 | 90 | f | 19 | 19 | 24 |
| 13 | 80 | f | 12 | 12 | 12 |
| 14 | 82 | f | 18 | 18 | 21 |
| 15 | 80 | f | 20 | 20 | 22 |

Control group

| Subject | Age | Gender | start | Six months | Twelve months |
|---|---|---|---|---|---|
| 1 | 72 | m | 29 | 29 | 29 |
| 2 | 79 | f | 19 | 17 | 16 |
| 3 | 90 | m | 29 | 28 | 27 |
| 4 | 88 | m | 18 | 17 | 14 |
| 5 | 82 | f | 17 | 15 | 13 |
| 6 | 82 | m | 12 | 11 | 8 |
| 7 | 71 | m | 32 | 32 | 29 |
| 8 | 90 | f | 18 | 17 | 13 |
| 9 | 90 | m | 29 | 29 | 26 |
| 10 | 78 | f | 23 | 22 | 17 |
| 11 | 89 | f | 14 | 11 | 7 |
| 12 | 93 | f | 24 | 22 | 20 |
| 13 | 81 | m | 20 | 20 | 21 |
| 14 | 87 | m | 17 | 16 | 17 |
| 15 | 77 | f | 25 | 26 | 25 |

2.1.1) Analysis with all the individuals

In the comparison carried out taking all the individuals into account, the mean difference between the first (start) and the second measurements (six months) of the individuals using the system is *x̅* = -0.1±1.1 (slight fall in the scores) whilst for the control individuals, the mean modifications were of *x̅* = -0.9±1.0 (greater fall in scores). The comparison of the medians with the Mann-Whitney U test produces statistically significant differences (p = 0.023), which is why the null hypothesis that there is no difference in the score modifications between both groups is rejected. In any case, the qualitative analysis (improvement vs. no improvement) does not detect significant differences (p>0.05; Fisher's exact test).

| | Exper. group | Control group |
|---|---|---|
| Samples | 15 | 15 |
| Average age | 83.8±5.7 | 83.3±6.9 |
| Average score at the start | 17.4±5.0 | 21.7±6.1 |
| | | |
| 6 months of sessions | | |
| Average modification of score | -0.1 | -0.9 |
| Standard deviation | 1.1 | 1.0 |
| Median | 0.0 | -1.0 |
| IQR | 0.0 | 1.5 |
| Mann-Whitney U test (p) | 0.023 | |
| Fisher's exact test (p) | 0.598 | |
| | | |
| 12 months of sessions | | |
| Average modification of score | 1.2 | -2.9 |
| Standard deviation | 2.7 | 2.3 |
| Median | 2.0 | -3.0 |
| IQR | 2.0 | 3.0 |
| Mann-Whitney U test (p) | <0.001 | |
| Fisher's exact test (p) | <0.001 | |

In contrast, the differences after 12 months of sessions (start-12 months) are highly significant for both the quantitative and qualitative analyses. Thus, the mean difference between the first and the third measurement of the individuals using the system *x̅* = = 1.2±2.7 indicates an increase in the scores, with a median (±IQR) of Me = 2.0±2.0; whilst for the control individuals, the mean modifications were *x̅* = -2.9±2.3 (reduction in scores) and Me = -3.0±3.0. The comparison of the medians with the Mann-Whitney U test produces statistically significant differences (p<0.001), similarly to Fisher's exact test (p<0.001), such that it follows that there are significant differences in the score modifications between both groups and that the system of the present invention improves the scores of the individuals treated. After 12 months of applying the system, the percentage of individuals that improved was 73.3%, whilst in the control group this value reduced to 6.7%.

| | Exper. group | Control group |
|---|---|---|
| Samples | 15 | 15 |
| | | |
| After 6 months | | |
| Users that improve | 20.0% | 6.7% |
| Users that do not improve | 80.0% | 93.3% |
| Total | 100.0% | 100.0% |
| | | |
| After 12 months | | |
| Users that improve | 73.3% | 6.7% |
| Users that do not improve | 26.7% | 93.3% |
| Total | 100.0% | 100.0% |

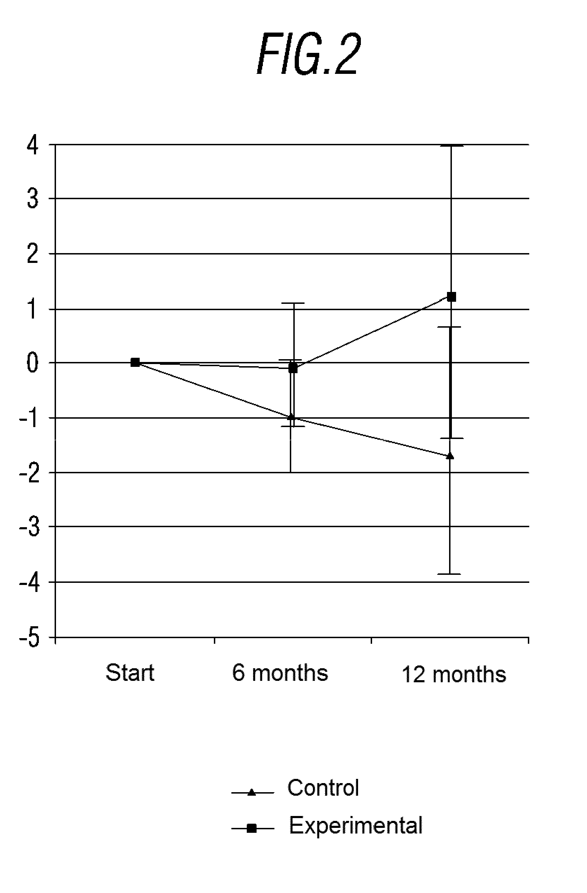

Thus, it is possible to confirm that this improvement is more pronounced after 12 months of use. In figure 2, it is possible to observe changes in the mean scores and their standard deviations in the two groups after 6 and 12 months of use.

Whilst deterioration is progressive in individuals that do not use the system, the use of the system stops deterioration in the first six months and entails an improvement after 12 months of use.

### 2.1.2) Analysis by gender

After having confirmed that use of the system results in an increase of the MMSE scores, it was of interest to test whether there are differences based on the gender of the individual. In this analysis, we are faced with the disadvantage that we only have three male individuals in the experimental group, thus meaning that the results obtained for this group will hardly offer significant differences, which means that a larger sample will be needed in future. In any case, similarly to the general sample, the scores of the men reduce after six months of treatment (*x̅* = -0.7±1.2) but increase similarly to the mean obtained with all the individuals grouped after 12 months of use (*x̅* = 1.0±3.6). The men in the control group presented a progressive decrease in their scores with a mean of *x̅* = -0.5±0.5 after 6 months and *x̅* = -1.9±2.0 after twelve months. None of the comparisons offered significant differences (p > 0.05).

| | Men | | Women | |
|---|---|---|---|---|
| | Exper. group | Control group | Exper. group | Control group |
| Samples | 3 | 8 | 12 | 7 |
| | | | | |
| 6 months of use | | | | |
| Average modification of score | -0.7 | -0.5 | 0.0 | -1.4 |
| Standard deviation | 1.2 | 0.5 | 1.0 | 1.3 |
| Median | 0.0 | -0.5 | 0.0 | -2.0 |
| IQR | 1.0 | 1.0 | 0.3 | 1.0 |
| Mann-Whitney U test (p) | 1.000 | | 0.028 | |
| Fisher's exact test (p) | N/A | | 0.619 | |
| | | | | |
| 12 months of use | | | | |
| Average modification of score | 1.0 | -1.9 | 1.3 | -4.1 |
| Standard deviation | 3.6 | 2.0 | 2.7 | 2.3 |
| Median | 2.0 | -2.5 | 2.0 | -4.0 |
| IQR | 3.5 | 3.3 | 1.5 | 2.0 |
| Mann-Whitney U test (p) | 0.194 | | 0.001 | |
| Fisher's exact test (p) | 0.152 | | 0.001 | |

Since the groups included a greater number of women than men, the results obtained for women offer more solid conclusions. The system applied to the experimental group shows that women stop deteriorating after 6 months of use (*x̅* 0.0±1.0) and improve after 12 months of use (*x̅* = 1.3±2.7). In contrast, deterioration in the control group was accentuated, with a mean of *x̅* = -1.4±1.3 at 6 months and *x̅* = - 4.1±2.3 at 12 months. Although the volume of data is not very large, a deterioration is noted in women not using the system of approximately double that of men in the same time period. The differences observed are statistically significant after 12 months of use (p = 0.001; Mann-Whitney U test; p = 0.001; Fisher's exact test), although if only the first six months of use are compared, the differences are only seen in the quantitative analysis (p = 0.028; Mann-Whitney U test; p = 0.619; Fisher's exact test). The improvement due to the system in both men and women is observed mainly after 12 months, with improvement percentages between 66.7% in men treated and 75% in women, whilst the improvement percentages in the control group are of 12.5% in men and none in women.

| | Men | | Women | |
|---|---|---|---|---|
| | Exper. group | Control group | Exper. group | Control group |
| Samples | 3 | 8 | 12 | 7 |
| | | | | |
| After 6 months | | | | |
| Users that improve | 0.0% | 0.0% | 25.0% | 14.3% |
| Users that do not improve | 100.0% | 100.0% | 75.0% | 85.7% |
| Total | 100.0% | 100.0% | 100.0% | 100.0% |
| | | | | |
| After 12 months | | | | |
| Users that improve | 66.7% | 12.5% | 75.0% | 0.0% |
| Users that do not improve | 33.3% | 87.5% | 25.0% | 100.0% |
| Total | 100.0% | 100.0% | 100.0% | 100.0% |

### 2.1.3) Analysis by age

Similarly to the analysis by gender, it was also of interest to know how effective the present system was in relation to the age of the users. For this analysis, we divided the samples into two groups, one made up of people between the ages of 70 and 85 and the other of people with an age comprised between 85 and 96. In this case, the groups are better compensated than in the analysis by gender with at least 5 individuals in each subgroup.

| | Aged 70-85 | | Aged 86-96 | |
|---|---|---|---|---|
| | Exper. group | Control group | Exper. group | Control group |
| Samples | 10 | 8 | 5 | 7 |
| | | | | |
| 6 months of use | | | | |
| Average modification of score | -0.3 | -0.6 | 0.2 | -1.3 |
| Standard deviation | 1.3 | 1.1 | 0.4 | 1.0 |
| Median | 0.0 | -0.5 | 0.0 | -1.0 |
| IQR | 0.0 | 1.3 | 0.0 | 0.5 |
| Mann-Whitney U test (p) | 0.460 | | 0.010 | |
| Fisher's exact test (p) | 1.000 | | 0.417 | |
| | | | | |
| 12 months of use | | | | |
| Average modification of | 0.4 | -2.4 | 2.8 | -3.6 |
| Standard deviation | 2.8 | 2.4 | 1.9 | 2.2 |
| Median | 1.5 | -3.0 | 3.0 | -4.0 |
| IQR | 1.8 | 4.0 | 2.0 | 2.0 |
| Mann-Whitney U test (p) | 0.021 | | 0.003 | |
| Fisher's exact test (p) | 0.025 | | 0.010 | |

In this analysis, in general no differences were observed in the scores at six months of use for either of the two groups (p > 0.05), except for the Mann-Whitney test in the oldest group (age 86-96, p = 0.010; Mann-Whitney U test). In contrast, the modifications at 12 months of use of the system are substantial, all the tests being statistically significant both for the age 70-85 group (p = 0.021; Mann-Whitney U test; p = 0.025; Fisher's exact test) and the age 86-96 group (p = 0.003; Mann-Whitney U test; p = 0.010; Fisher's exact test). It must be noted that, as anticipated, the individuals in the control group deteriorate quicker at an older age, with a mean reduction in scores at 6 months of *x̅* = -0.6±1.1 for the age 70-85 and *x̅* = -1.3±1.0 for the age 86-96, this tendency being maintained at 12 months (*x̅* = -2.4±2.4 at ages 70-85 and *x̅* = -3.6±2.2 at ages 86-96). Surprisingly, the system appears to have a greater effect on older users since at 12 months of use, the increase in scores for the ages 70-85 group was *x̅* = 0.4±2.8, whilst in the ages 86-96 group it was *x̅* = 2.8±1.9. Even at 6 months of use, the scores for the ages 86-96 group were greater (*x̅* = 0.2±0.4) than for the ages 70-85 group (*x̅* = -0.3±1.3), in which a slight decrease may be observed. Although the scores increase more for older individuals, the improvement percentages are similar in both user groups (70% and 80%). This result enables us to differentiate between the quantity of individuals that improve and the quality of improvement of the individuals.

| | Aged 70-85 | | Aged 86-96 | |
|---|---|---|---|---|
| | Exper. group | Control group | Exper. group | Control group |
| Samples | 10 | 8 | 5 | 7 |
| | | | | |
| After 6 months | | | | |
| Users that improve | 20.0% | 12.5% | 20.0% | 0.0% |
| Users that do not improve | 80.0% | 87.5% | 80.0% | 100.0% |
| Total | 100.0% | 100.0% | 100.0% | 100.0% |
| | | | | |
| After 12 months | | | | |
| Users that improve | 70.0% | 12.5% | 80.0% | 0.0% |
| Users that do not improve | 30.0% | 87.5% | 20.0% | 100.0% |
| Total | 100.0% | 100.0% | 100.0% | 100.0% |

### 2.1.4) Analysis based on the level of initial deterioration

Finally, it was also of interest to analyse the evolution of the users taking into account their level of initial deterioration into account. In order to do so, individuals were divided into two groups, those with an initial score (June 2012) equal to or lower than 18 and, on the other hand, those with more than 18 points. Similarly to the analysis by age, in this case no differences were observed in the scores at 6 months of use for either of the two groups (p > 0.05), except for the Mann-Whitney test for the group with initial scores equal to or lower than 18 (p = 0.008; Mann-Whitney U test), which was probably due to the rapid deterioration that occurred in this control group. The modifications in the scores at 12 months of use are statistically significant for both the group with low initial scores (p = 0.021; Mann-Whitney U test; p = 0.028; Fisher's exact test) and the group with high scores (p = 0.008; Mann-Whitney U test; p = 0.011; Fisher's exact test). In this analysis, it was observed that the improvement of scores at 12 months of use are greater (approximately double) when the initial scores are greater than 18 (*x̅* = 1.8±2.6) than when they are lower (*x̅* = 0.8±2.9). The same occurs in the control group, with double the deterioration in individuals with lower scores (*x̅* = - 4.0±2.3) than in individuals with initial scores over 18 (*x̅* = -2.2±2.2).

| | Initial score ≤ 18 | | Initial score > 18 | |
|---|---|---|---|---|
| | Exper. group | Control group | Exper. group | Control group |
| Samples | 9 | 6 | 6 | 9 |
| | | | | |
| 6 months of use | | | | |
| Average modification of score | -0.2 | -1.5 | 0.0 | -0.6 |
| Standard deviation | 1.1 | 0.8 | 1.1 | 1.0 |
| Median | 0.0 | -1.0 | 0.0 | 0.0 |
| IQR | 0.0 | 0.8 | 0.8 | 1.0 |
| Mann-Whitney U test (p) | 0.008 | | 0.328 | |
| Fisher's exact test (p) | 1.000 | | 0.525 | |
| | | | | |
| 12 months of use | | | | |
| Average modification of score | 0.8 | -4.0 | 1.8 | -2.2 |
| Standard deviation | 2.9 | 2.3 | 2.6 | 2.2 |
| Median | 1.0 | -4.0 | 2.0 | -3.0 |
| IQR | 2.0 | 0.8 | 0.8 | 3.0 |
| Mann-Whitney U test (p) | 0.012 | | 0.008 | |
| Fisher's exact test (p) | 0.028 | | 0.011 | |

Just like the previous analyses, the improvement percentages are greater in the experimental groups, and are more prominent after 12 months of use.

| | Initial score ≤ 18 | | Initial score > 18 | |
|---|---|---|---|---|
| | Exper. group | Control group | Exper. group | Control group |
| Samples | 9 | 6 | 6 | 9 |
| | | | | |
| After 6 months | | | | |
| Users that improve | 11.1% | 0.0% | 33.3% | 11.1% |
| Users that do not improve | 88.9% | 100.0% | 66.7% | 88.9% |
| Total | 100.0% | 100.0% | 100.0% | 100.0% |
| | | | | |
| After 12 months | | | | |
| Users that improve | 66.7% | 0.0% | 83.3% | 11.1% |
| Users that do not improve | 33.3% | 100.0% | 16.7% | 88.9% |
| Total | 100.0% | 100.0% | 100.0% | 100.0% |

### 2.2) Conclusions

When all the samples are analysed at six months of use, the data distribution in the two groups are already different, although they are not when they are dealt with qualitatively. At 12 months of use, the differences are statistically significant, whatever the statistical approach used.

When the results are analysed by gender, it is observed that the results for women are similar to those detected in the total sample with an increase in scores due to use, whilst no significant increase is observed in men. In this last case, the lack of significance seems to be caused by the small sample size, since there were only three men in the experimental group.

When we divide the individuals by age, the results indicate that the use of the system has a greater effect on those older than 85 than those that are younger. This effect seems to be more of a quality improvement than an improvement the quantity of individuals that improve, since although the quantity of individuals that improve is similar in both groups, those older than 85 show a greater mean increase in scores that is six times greater after 12 months of use.

Lastly, regarding the categorisation of the individuals based on an initial score less than or greater than 18, the results suggest a greater effect of the system on individuals with higher initial scores.

The details, shapes, dimensions and other accessory elements, as well as the materials used to manufacture the system for cognitive and sensory stimulation and the method followed by the same of the invention, may be suitably substituted for others that do not diverge from the scope defined by the claims included below.

## Claims

1. A system for cognitive and sensory stimulation (1), **characterised by** the fact that it comprises a processing unit (11) in data communication with display means (21), at least one user interface (22) and sensory stimulation means, the processing unit (11) being configured to store and manage a plurality of operating sequences from the sensory stimulation means based on a number of signals received from the user interface (22) and the cognitive and sensory interaction with a user, in which the sensory stimulation means are configured to stimulate at least one visual, tactile, olfactory and sound field of the user or a combination thereof.

2. A system for cognitive and sensory stimulation (1), **characterised by** the fact that it comprises a processing unit (11) in data communication with display means (21), at least one user interface (22) and sensory stimulation means, the processing unit (11) being configured to store and manage a plurality of operating sequences from the sensory stimulation means based on a number of signals received from the user interface (22) and cognitive or sensory interaction with a user, wherein the sensory stimulation means are configured to stimulate at least one visual, tactile, olfactory or sound field of the user or a combination of thereof.

3. The system for cognitive and sensory stimulation (1), according to any of the preceding claims, wherein the display means (21) and the user interface (22) are incorporated into the same touchscreen.

4. The system for cognitive and sensory stimulation (1), according to claim 3, wherein the touchscreen is arranged on a table structure (23).

5. The system for cognitive and sensory stimulation (1), according to any of the preceding claims, wherein the sensory stimulation means comprise a water column (3) linked to at least one bubble generator (31), a first coloured light emitter (32) and a first button panel (33).

6. The system for cognitive and sensory stimulation (1), according to any of the preceding claims, wherein the sensory stimulation means furthermore comprise a second light emitter (4), which is configured to emit different colours, said second light emitter (4) being linked to a second button panel (41).

7. The system for cognitive and sensory stimulation (1), according to any of the preceding claims, wherein the sensory stimulation means furthermore comprise at least one long, flexible element, which is configured to emit light and is arranged on a surface where the user is positioned.

8. The system for cognitive and sensory stimulation (1), according to any of the preceding claims, wherein the sensory stimulation means furthermore comprise at least one main projector (6) for changeable visual effects.

9. The system for cognitive and sensory stimulation (1), according to any of the preceding claims, wherein the sensory stimulation means furthermore comprise a light-up scale (7) linked to at least one microphone.

10. The system for cognitive and sensory stimulation (1), according to any of the preceding claims, wherein the sensory stimulation means furthermore comprise at least one aroma diffuser (75).

11. The system for cognitive and sensory stimulation (1), according to any of the preceding claims, wherein the sensory stimulation means furthermore comprise sound emitting means.

12. The system for cognitive and sensory stimulation (1), according to claim 11, wherein the sensory stimulation means furthermore comprise a vibrating water bed (9) linked to at least one water heater.

13. A method for cognitive and sensory stimulation that may be applied in the cognitive and sensory stimulation (1), according to any of the preceding claims, **characterised by** the fact that it comprises the stages of:
a) entering external qualification data into the user interface (22);
b) linking the external qualification data to a plurality of indicators through the processing unit (11);
c) quantifying the external qualification data for each indicator through the processing unit (11);
d) determining the operating sequence of the sensory stimulation means according to the previous stages through the processing unit (11) and activating the sensory stimulation means; and
e) determining the cognitive stimulation exercises to be carried out by the user through the processing unit (11) and activating the user interface (22) in order to carry out the cognitive stimulation exercises.

14. The method for cognitive and sensory stimulation, according to claim 13, wherein in stage a) the indicators are at least one from a cognitive level, sensory state, functional level, behavioural level, level of awareness and level of consciousness or a combination of the same.

15. The method for cognitive and sensory stimulation, according to claim 14, wherein stage d) depends on at least one of the indicators for behavioural level, level of awareness, level of consciousness and sensory state or a combination of the same.

16. The method for cognitive and sensory stimulation, according to claim 14, wherein stage e) depends on at least one of the indicators from a cognitive level and functional level or a combination of the same.

17. The method for cognitive and sensory stimulation, according to any of claims 13-16, wherein the operation of at least one of the sensory stimulation means and the activation of the user interface (22) to carry out the cognitive stimulation exercises is carried out together and in a coordinated manner or sequentially.

18. The method for cognitive and sensory stimulation, according to claims 13-17, wherein it furthermore comprises storing a set of execution data obtained once stages d) and e) have been carried out and linking said execution data to a user through the processing unit (11).
